# EUROPEAN PATENT APPLICATION

(11) **EP 2 381 247 A1**
(43) Date of publication of application: **26.10.2011**
(21) Application number: 11162899.6
(22) Date of filing: 18.04.2011
(51) Int. Cl.: G01N 23/04, G01N 33/46

(54) **Method and apparatus for scanning the internal quality of logs**

(30) Priority: 20.04.2010 IT VR20100077
(71) Applicant: MICROTEC S.r.l., 39042 Bressanone (Bolzano) (IT)
(72) Inventor: Giudiceandrea, Federico, 39042, Bressanone (Bolzano) (IT)
(74) Representative: Ponchiroli, Simone

(57) **Abstract**

Method for scanning the internal quality of logs envisaging the irradiation of at least a longitudinal section (4) of a log (1) with a beam (2) of electromagnetic radiations while this is made to move forward axially along a movement trajectory while at the same time creating a relative helical movement between the log (1) and the beam (2) of electromagnetic radiations. For a plurality of distinct positions of the log (1) the residual intensity is detected of the electromagnetic radiations that have crossed the longitudinal section (4) and the reciprocal position and spatial orientation of the longitudinal section (4) itself and the beam (2). The reciprocal position is then determined of the longitudinal sections of the log (1) in relation to which the residual intensity has been detected and the internal conformation can be reconstructed of the log (1) by means of a tomographic processing of the residual intensities detected for each position of the log (1), wherein account is taken of the reciprocal arrangement of the various longitudinal sections detected the one to the other and with respect to the beam (2).

## Description

The subject of the present invention is a method for scanning the internal quality of logs by means of a tomographic type procedure.

Currently, to scan the internal quality of logs, two main systems exist: the rotating tomographic system and the fixed multiple source system.

In the first case, the tomography revolves around the log (approximately around its main axis) and each cross "slice" of the log undergoes a plurality of scans according to different angular positions (normally to obtain good information, the scans have to be repeated for a little more than half a turn around the log). Generally speaking, furthermore, the tomography detector consists of a matrix detector having one or more rows of sensors (the rows of sensors extend crossways to the main axis and are positioned alongside one another along the main axis). Consequently, each longitudinal section of the analyzed log (the length of which equals the width of the electromagnetic beam that crosses it), is split into one or more elementary slices, each of which has a width substantially equal to the width of a row of scanning sensors.

The reconstruction of the appearance of the slice is done by means of known algorithms widely described in scientific literature and therefore not described in any further detail here.

The resolution of the obtained information depends on the size of the sensors (in an inversely proportionate way), on their number (in a directly proportionate way) and on the number of scans made.

In a known way in fact, the tomographic reconstruction is carried out by virtually splitting up the volume of the slice being examined into a plurality of elementary volumes where density is taken as constant. Each volume has a dimension on the position plane of the beam of electromagnetic radiations which depends on the number of sensors, on their size and on the number of scans, and length equal to the width of the relative row of sensors.

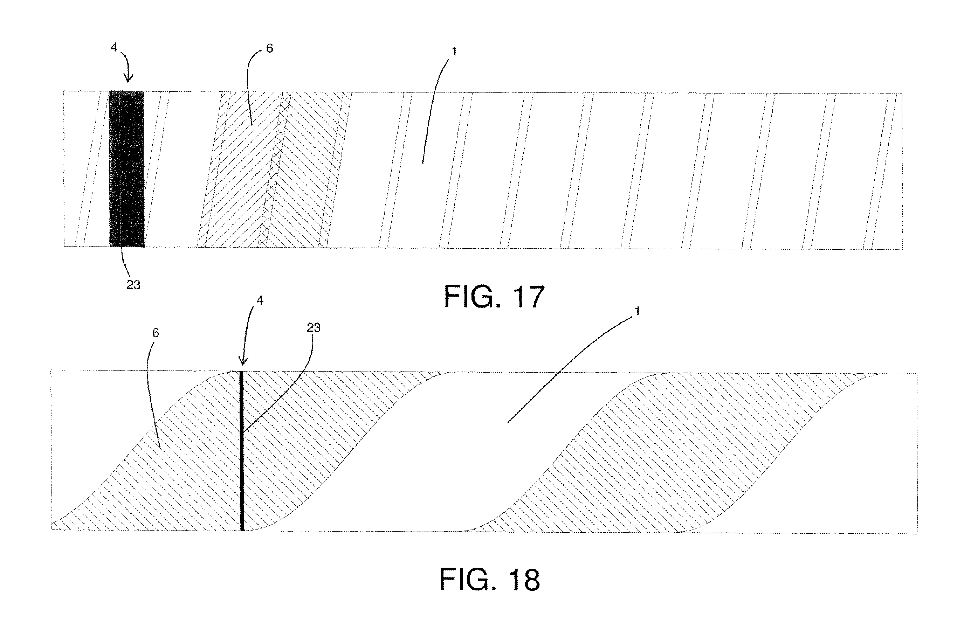

In point of fact, furthermore, to speed up scanning operations, the rotation of the tomography occurs at the same time as the longitudinal forward movement of the log. The two movements are in fact synchronised so that each elementary volume is detected by at least one sensor for the required number of distinct angular positions. This situation is schematized in figure 17 which shows a side view of a log on which are drawn the instantaneous position of the electromagnetic beam (black rectangle) and the spirals described by the projection of such beam detected on the surface of the log. The two areas outlined by the dotted line also indicate the width of the spiral and show how, at each turn, there is a certain superimposition of the detected areas.

The rotating tomographs thus allow obtaining a high resolution of details but call for very long scanning times to examine a whole log slice by slice, because of the need to move the beam emitter and the detector in a controlled and precise way around the log, which must be made to move forwards very slowly, without any accidental movements.

An example of rotating tomograph is disclosed in patent application WO 02/091286; in the apparatus described in that patent application, in fact, the tomograph rotates around the log which is made to move forwad longitudinally. In particular, in order to allow that system to properly work, the log is made to move forward in a controlled way (the log is mechanically pusher from the back) with its longitudinal axis always perfectly aligned along a straight line. In other words, in that apparatus any displacement of the log in the plane transversal to the moving forward direction is avoided. The second system currently used (described for example in US patent 5,023,805) always envisages on the other hand the log being analyzed slice by slice, but by means of a plurality of fixed sources (and corresponding detectors) distributed around the log itself.

In this case, operation is the same as that of a rotating tomography which, for each slice of the log, makes a number of scans equal to the number of fixed sources, whenever it finds itself in the angular position corresponding to each of them.

This system is very definitely faster than the previous one but its resolution is not of the best as it allows detecting only some types of well-localised faults. In particular, this system is unable to detect the presence of cracks in the log. Such defect in fact can be detected by means of a tomographic scan only if observed along its direction of extension, while it is almost invisible if observed in a cross direction (in view of its very low thickness, the difference in the attenuation of the electromagnetic radiations is practically irrelevant compared to a log without a crack).

In the case of this second known system too, the movement of the log through the tomography must be precisely controlled to avoid any side movement that would affect the tomographic result. Consequently, it is not in any case possible to examine the logs considering the forward movement speed typical of timber working plants.

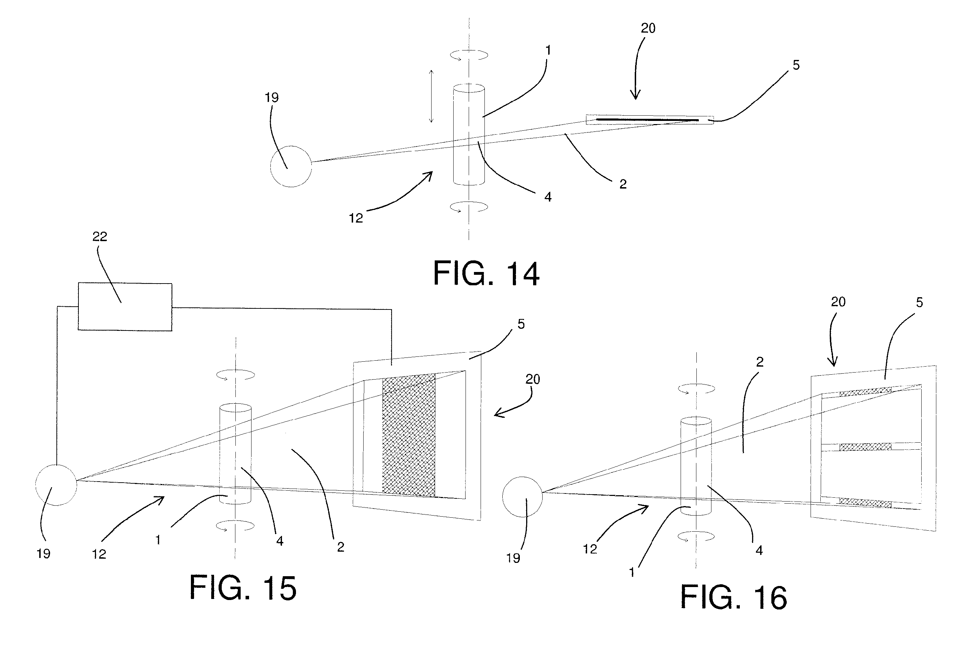

In medical environments, relatively faster rotating tomographs are built that exploit the so-called cone-beam method (conical beam, meaning diverging starting from the source) which uses very large sensor matrices to be able to move forward quickly. This method is schematized in figure 15, where a log-shaped body, an emitter, a flat sensor, the conical beam and the image of the body can be seen. Special logarithms then allow deciphering the image, offsetting the different inclination (and therefore the different quantity of material crossed) of the beams detected by the different rows of sensors. The figure 16 shows a variation of the cone-beam method wherein only some parts of the cone of electromagnetic radiations are detected.

Finally, the figure 14 shows, instead, the so-called fan-beam method (this too used in the wood industry) wherein the electromagnetic beam is perpendicular to the axis of the log and is of limited thickness. In the figure 14, in fact, the detector has a substantially linear extension.

Not even these methods manage however to ensure the speeds required in the wood industry where the logs move forward at a speed of a few metres a second considering that in this case as well it is envisaged that the examined object must not undergo accidental movements.

Finally, it must be remembered that numerous mathematic studies have been developed in relation to the problems tied to the tomography sector. On the basis of such studies, an identical number of specific algorithms have been developed for using the cone-beam technology wherein both the inclination of each beam with respect to the log and the distance between the source of electromagnetic radiation and the log itself must be taken into consideration. In this sector, among the most recent studies must be mentioned that described in the article M. Kapralov, A. Katsevich - "A Study of 1PI Algorithms for a General Class of Curves", SIAM J. IMAGING SCIENCE, Vol. 1, No. 4, pages 418-459, wherein is examined the problem of the movement of the emitter-detector unit with respect to an object according to an irregular spiral trajectory.

In this situation, the technical task at the bottom of the present invention is to develop a method for scanning the internal quality of logs that eliminates the aforementioned drawbacks.

In particular, object of the present invention is to develop a method for scanning the internal quality of logs that permits achieving high productivity. In particular, the technical task of the present invention is to develop a method for scanning the internal quality of logs that permits examining the logs without slowing down their forward movement along the working line. The specified technical task and the indicated objects are substantially achieved by a method for scanning the internal quality of logs, according to what is described in the attached claims.

Further characteristics and advantages of the present invention will appear clearer from the detailed description of a number of preferred, albeit not exclusive embodiments of a method for scanning the internal quality of logs, illustrated with reference to the attached drawings, wherein:
- figure 1 shows a schematic side view, with a number of parts removed to better highlight others, of an appliance for implementing the method forming the subject of the present invention;
- figure 2 shows the appliance of figure 1 in schematic view from above;
- figure 3 shows the appliance of figure 1 in schematic front view (from the left);
- figure 4 schematically shows an operating phase which can be performed according to the method forming the subject of the present invention;
- the figures from 5 to 6 show three possible profiles detectable during the operating phase shown in figure 4;
- the figures from 8 to 13 show some possible variations of the operating phase of figure 4;
- the figures from 14 to 16 show three possible embodiments of the method forming the subject of the present invention also common to the state of the art; and
- the figures from 17 to 19 schematically show in graphic form the pattern of an operating phase of the method forming the subject of the present invention in three different embodiments.

In the attached illustrations, the reference number 1 always identifies a log to which can be applied the method forming the subject of the present invention.

The method for scanning the internal quality of logs forming the subject of the present invention is based, similarly to known methods, on a tomographic examination combined with a relative helical movement between the beam 2 of electromagnetic radiations and the log 1.

In agreement with the present invention in fact, a log 1, with an own prevalent direction of extension identifiable with its main extension axis 3, is taken and made to move forward along a movement trajectory with its axis arranged substantially parallel to the movement trajectory itself. At the same time, a relative helical movement is created between the beam 2 of electromagnetic radiations and the log 1. In the preferred embodiment, the helical movement is created by rotating the log 1 around an instantaneous rotation axis substantially parallel to the movement trajectory (and therefore around the main extension axis 3). In other embodiments, in terms of this aspect identical to the known systems, it may on the other hand be envisaged to have the beam 2 of electromagnetic radiations (and therefore advantageously, the means of emission and detection of such beam) substantially rotate around the log 1 with respect to the same axis indicated above. In both cases, in any case, the axial component of the helical movement is advantageously ensured by the forward movement of the log 1 along the movement trajectory.

During the forward movement, in any case, the position of the log 1 is monitored to determine where it is located along the movement trajectory. This can be done in many different ways. Nevertheless, due to the possibility of sliding occurring, it is best to use direct controls without contact rather than encoders. Advantageously, for example, optical sensors or laser detectors with Doppler effect can be used (such as those called LS9000 made by the US company Beta LaserMike).

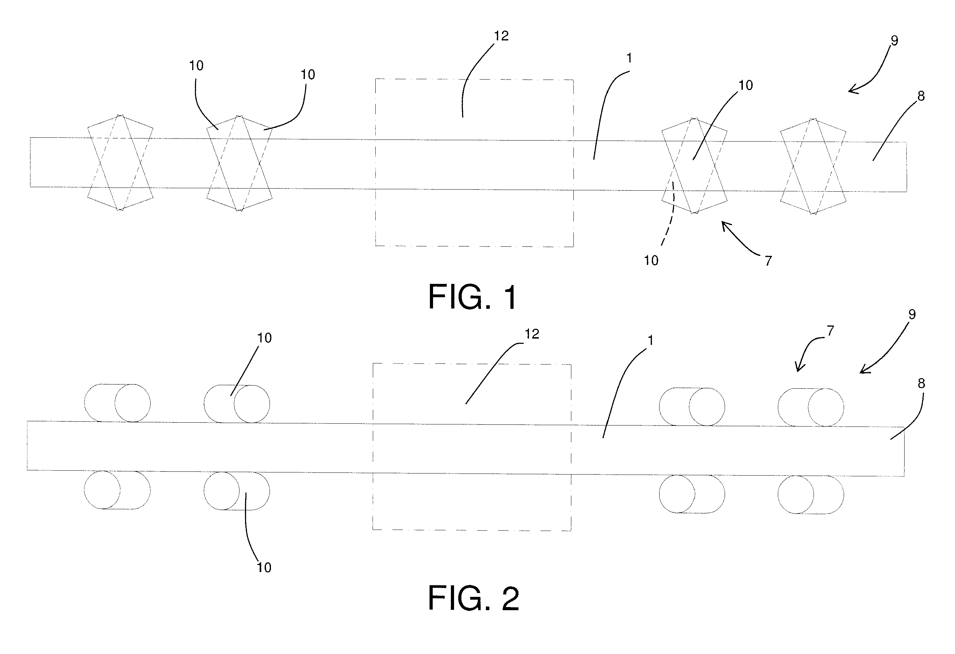

During helical movement, at least a longitudinal section 4 of the log 1 is expected to be irradiated with at least a beam 2 of electromagnetic radiations, directed in one or more directions of irradiation transversal to the main extension axis 3, according to the common tomographic operating modes. Advantageously, the irradiation occurs in a continuous way in correspondence to an area of irradiation (in figure 1 preferably placed inside the dash-dot rectangle) through which gradually passes the entire log 1 (as shown for example in the figures from 14 to 16). In other embodiments, in any case, the irradiation can also involve several separate portions of the log 1 which, for purposes of the present invention, can be interpreted both as different longitudinal sections, and as spaced sub-sections of a single longitudinal section 4.

Similarly to the known methods, the detection is also envisaged, by means of at least a detector 5 consisting of a plurality of sensors (preferably matrix sensors), of the residual intensity of the electromagnetic radiations which have crossed the longitudinal section 4 of the log 1 affected by the electromagnetic beam 2, and its repetition for a plurality of distinct positions of the log 1 itself. In point of fact, this is made concrete by repeating the scan at preset time intervals during which the log 1 moves forward by a certain amount (at the same time, there is also a certain relative rotation). Advantageously, furthermore, a beam 2 can be used generated according to the cone-beam method (figure 15 or 16).

In the illustrated embodiment, which envisages the rotation of the log, furthermore, the beam 2 of radiations is generated so as to be substantially at a standstill in space.

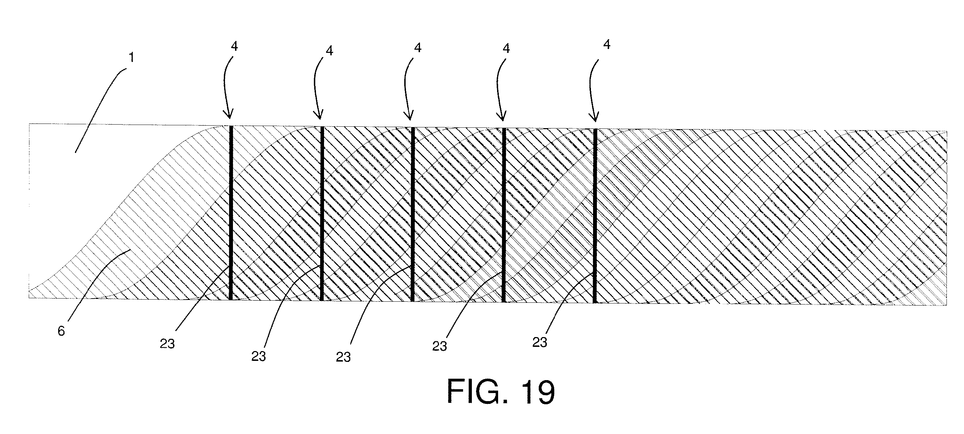

The figures from 17 to 19 show, on the one hand (coloured in black) the projection 23 on the surface of the log 1, according to the direction of irradiation of the section/s involved in an instant by the electromagnetic beam 2, and on the other, the surface beam/s 6 described by such projections following the helical movement relating to the log 1 and to the beam 2. In particular, in the case of figure 17, the helical movement is built up (as ratio between the forward speed and the rotation speed) in such a way that the spiral described by the projections of the irradiated sections covers the entire side surface of the log 1, partially superimposing on itself. In this embodiment, each internal point of the log 1 is observed for more than half a turn and an accurate tomographic reconstruction can be made of the internal structure. In the case of figure 18, on the other hand, the spiral does not cover the entire surface of the log 1 but only one of its bands (wide spiral), in agreement with what is provided in the Italian patent application no. VR2009A000146 in the name of this same applicant. To this same patent application also refers the solution of figure 19 where the irradiated longitudinal section 4 is split into five different sections the projections of which extend along five wide spirals partially superimposed the one on the other. The purpose of such methods is to allow detecting the presence of faults that extend axially, reducing tomographic processing to the utmost and increasing system speed as much as possible.

The present invention can in any case be applied indifferently to any type of tomographic reconstruction, including, for example, both complete tomographic reconstructions and tomographic reconstructions meant to detect only those characteristics having axial extension.

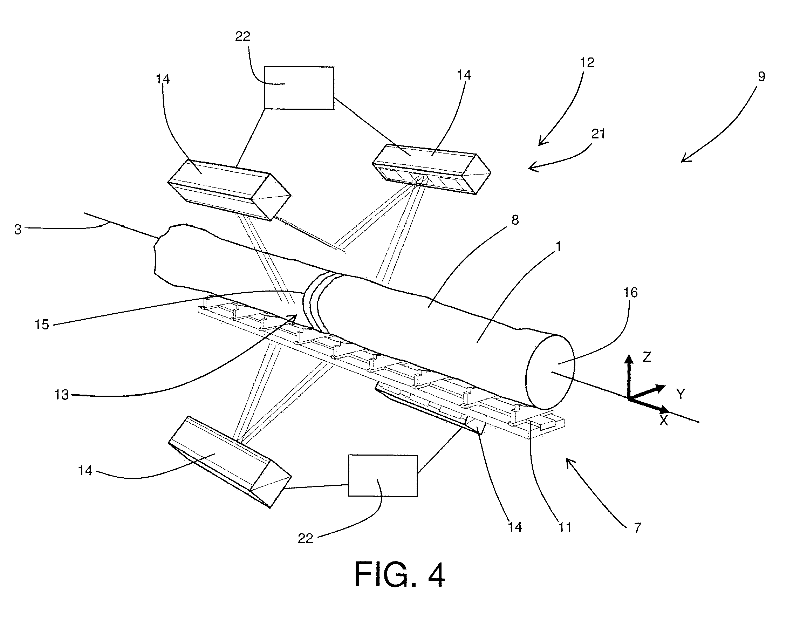

In the preferred embodiment, then, the log 1 is made to move forward and, if envisaged, to rotate around itself, with the support of supporting and movement means 7 which act on its side surface 8 (generally both under the log 1 and, in the case of the helical movement being obtained by means of a rotation of the log 1, sideways to this as shown in the figures from 1 to 4). The figures from 1 to 3 show an appliance 9 able to produce the helical movement while at the same time making the log 1 move forward and rotate. It comprises a plurality of pairs of counter-rotating rollers 10 arranged at the two sides of the log 1 and tilted in two opposite directions to convey at the same time to the log 1 both a force component along the direction of forward movement, and a rotational movement. Depending on requirements, then, the supporting and movement means 7 can also comprise or not a lower conveyor 11 as shown in the figure 4 (where, on the other hand, there are no rollers 10). Generally speaking, in any case, it is not envisaged that the conveyor be present in correspondence to the beam 2 so as not to alter the scan. Advantageously, the appliance 9 in figure 1 comprises four pairs of rollers 10 placed two upstream and two downstream with respect to a central detection area 12 (indicated by the dot-dash rectangle), in correspondence to which the phases described below are performed. This way in fact, the log 1 is always perfectly supported, even when its extremities are examined.

As has already been said, the log 1 moves forward, and if necessary rotates, thanks to the interaction of the supporting and movement means 7 with the side surface 8. The latter however, as is known, always appears irregular. Consequently, because the supporting and movement means 7 are advantageously fixed in space (as absolute position compared to the movement trajectory even though, to ensure forward movement, and if necessary rotation, these can have parts that move on themselves such as rollers 10 or conveyor chains), and because at every moment the profile of the surface portion in contact with the supporting and movement means 7 changes, consequently the position of the main extension axis 3 of the log 1 can change continuously with respect to the supporting and movement means 7 themselves. Moreover, this way, the position of the main extension axis 3 also varies with respect to the beam 2 of electromagnetic radiations. Furthermore, normally, during the movement of the logs 1, a lot of slipping occurs between the log 1 and the supporting and movement means 7, so the position of the log 1 with respect to the movement trajectory is never ever that foreseeable in theory.

In order to implement the idea behind the present invention of creating a relative helical movement between the log and the beam 2 of electromagnetic radiations, always usable in tomographic reconstruction, it was therefore necessary to tackle and overcome the problems just described.

In agreement with the present invention, the first step in obtaining this result was to determine, for each residual intensity scan (and therefore for each corresponding position of the log 1), the reciprocal position and spatial orientation of the longitudinal section 4 for which the residual intensity is detected and of the beam 2. Besides this, it was also envisaged to determine, at least implicitly, the reciprocal position of the longitudinal sections 4 of the log 1 in relation to which the residual intensity was detected of the electromagnetic radiations in the various positions.

In this respect, as will appear clearer below, it should be noticed that if two reference systems are assigned, a first to log 1 and a second to beam 2, following the forward movement of the log 1 itself, the first reference system integral with this can undergo, with respect to the second, not only a translation parallel to the movement trajectory and a rotation in the plane perpendicular to it, but also a series of further translations and rotations (e.g., due to the irregularity of the surface of the log 1 which interacts with the supporting and movement means 7). As is known in fact, any object that can move has, generally speaking, six degrees of freedom which, identifying a Cartesian reference system, can be identified as three possibilities of translation parallel to the three axes, and three possibilities of rotation around axes parallel to the Cartesian ones.

To better understand all this, it can therefore be useful to establish a Cartesian reference system of the type shown in figure 4, wherein the axis X corresponds to the direction of development of the movement trajectory, the axis Y to a direction (horizontal) perpendicular to it, and the axis Z to a direction (vertical) perpendicular to the horizontal plane position of axis X. Each movement of the log 1 can therefore be reconstructed as a series of translations and rotations with respect to the above three Cartesian axes. Consequently, if the log 1 were to merely move forward, its movement would follow that of the movement means and would only be describable as a translation parallel to the axis X.

In the case, on the other hand, of ideal helical movement obtained by means of a rotation of the log 1 on itself, its movement would only be describable as a combination of a translation parallel to the axis X and a rotation around it.

Nevertheless, as has been said, during the real forward movement, the log 1 can also move with respect to the supporting and movement means 7 according to the other degrees of freedom.

Generally speaking, the determination of the reciprocal position and spatial orientation of the various longitudinal sections and of the beam can occur using any procedure suitable for the purpose. By way of example, the position can be detected of one or more reference elements applied to the log. In the preferred embodiments in fact, the position of the beam is always known either because the beam is stopped or because its rotation is controlled with precision. In other words, only the log can undergo accidental movements.

In the preferred embodiments, however, the determination of the spatial position and orientation envisages the detection of the surface conformation and of the spatial position of at least a surface portion 13 of the log 1. Within the ambit of the present invention, by surface conformation is meant the extension in space of the surface portion 13 of the log 1 detected with reference to a reference system outside the log 1 itself (in practice normally integral with the means of detection and therefore with the supporting and movement means 7). By surface portion 13 on the other hand is meant a series of real points of the surface of the log 1 (the surface conformation of which represents the advancement in space).

In agreement with the present invention, the detection of the surface conformation of the log 1 must be performed in such a way that the surface portion 13 detected at each scan is at least in part superimposed on the surface portion 13 detected in at least another different scan.

This way, in fact, by comparing the surface conformation of a first portion detected at a certain time with that of a second portion partially superimposed on the first, detected at a subsequent time, it is possible to determine which movements the log 1 has undergone during the time between the switch from the first to the second position.

Figure 4 shows an example of appliance 9 for detecting the surface conformation of a log 1 arranged on supporting and movement means 7. In particular, in the drawing are shown four laser triangulation devices 14, each of which observes the log 1 in correspondence to three distinct equidistant planes perpendicular to its main extension axis 3. For each scan, the surface portion 13 consists of the series of the three circumferential profiles 15 thus detected. After detecting the surface conformation of a first portion therefore, it is enough to repeat the scan when the log 1 has moved forward by the distance between two planes to obtain a new surface portion 13 partially superimposed (by two circumferential profiles 15 out of three) on the first. After a further similar forward movement, a further surface portion 13 can be detected partially superimposed (this time by just one circumferential profile 15 out of three) on the first. And so on.

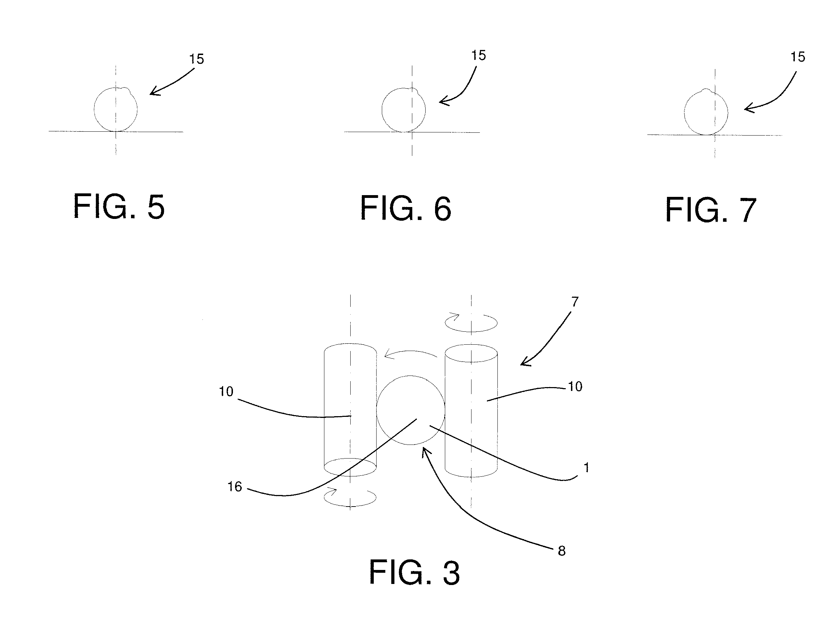

This situation is schematically identifiable in the figures from 5 to 7 where the case is represented of how the surface conformation of a same circumferential profile 15 could be detected in two subsequent scans (the dotted line represents the central plane of the profile 15 in the first scan). It can therefore be imagined that a certain profile 15 of the first detected portion be detected with the surface conformation indicated in figure 5. If, when this is detected in the scan of the second surface portion, the new scan were always represented by the figure 5, we could come to the conclusion that the second portion has not virtually moved with respect to the first except in axial direction X (e.g., it could have completed one or more turns around the axis X). We are therefore sure that the log 1 has altogether only undergone a translation along X.

If on the other hand, in the second scan, the profile 15 were to be detected as indicated in figure 6, we could come to the conclusion that the second portion, with respect to the first, has undergone one or more movements definable, as a whole, as a side translation (parallel to the axis Y in a reference system like that in figure 4).

A similar line of reasoning is possible in the case of the profile 15 being detected as shown in the figure 6 in the second scan. In this case however, the movement of the log 1 would not be a simple translation but the combination of a side translation and of a rotation around an axis perpendicular to the sheet (making reference to a Cartesian system as in figure 4, the translation would be parallel to the axis Y, while the rotation would be around an axis parallel to axis X).

As regards the procedures whereby the scans are made, these can be of any sort and are not described here in detail inasmuch as they are known. In the preferred embodiments, in any case, the scans are generally made by projecting a beam 2 of light (preferably laser) on the surface of the log 1 and more or less instantly detecting the surface conformation of the lit up area using the triangulation method. In other embodiments on the other hand, the scan is effected by detecting the texture of the surface of the log 1 (i.e., its outer appearance). Reconstructing the three-dimensional aspect of an irregular object is in fact known, on the basis of how the appearance varies of the same area of the surface of the object itself along with the variation of the reciprocal positioning between point of observation (detection) and detected surface area. In other embodiments, the scan of the surface of the log 1 can also be effected by means of the known method called "time of flight" wherein the position of the points of the surface is obtained by measuring the time taken by a light pulse to reach the log 1 and return to a detection sensor (this method is not described in detail here either because it is well known). Advantageously, to scan the entire surface of the log 1, rotating mirrors can be used combined with interferometric techniques.

As regards the phase of determining at least implicitly the reciprocal position of the longitudinal sections 4, in the preferred embodiment, this envisages, for each position of the log 1, comparing at least implicitly the surface conformation detected in that position, with a second surface conformation detected in correspondence to a different position of the log 1, and on the basis of such comparison determining at least implicitly the reciprocal position of the longitudinal sections of the log 1 in relation to which the residual intensity has been detected of the electromagnetic radiations in the two positions of the log 1. In order to do this, the compared surface conformations must correspond to portions of the surface of the log 1, which have at least two points in common, so that the comparison phase can be effected by virtually superimposing the common points of the two surface conformations detected as previously explained. Preferably, in fact, the comparison phase envisages comparing, for each position, the surface conformation detected in that position with the surface conformation detected in a different position, to determine the movements that have affected the log 1 in the switch from one position to the other. Such movements, when the position of the log 1 along the movement trajectory is known, can in fact be defined with reference to the five residual degrees of freedom.

Consequently, once the surface conformations have been superimposed, the transformation formulas are obtained that define the movement and which, because the log 1 is a rigid body, can be used in a reverse way to establish the reciprocal positions of the two corresponding longitudinal sections in correspondence to which the residual intensity of the electromagnetic radiations has been detected.

It must in any case be said that, in case of need, it can also be envisaged to compare, for each position, the surface conformation detected in a plurality of different positions. This can prove particularly useful in the case of the log 1 having a particularly uniform surface with few distinctive characteristics. Furthermore, the detection of the surface conformation can also be performed more frequently than the detection of the residual electromagnetic intensity. The less the movement affecting the log 1 in fact between the two surface conformation scans, the simpler it is to determine such movement. Consequently, a relatively large movement can be more easily and more precisely calculated as the sum of a lot of small movements.

The last general phase of the method forming the subject of the present invention finally envisages reconstructing the internal conformation of the log 1 by means of computerised tomographic processing (i.e., by means of calculation algorithms intended for such application) of the residual intensities detected for each position of the log 1. In doing this, the intensities detected are combined taking into account the reciprocal positions of the various longitudinal sections 4 the one to the other and with respect to the beam 2 and therefore, advantageously, on the basis of the movements of the log 1 calculated between each detection position. In the preferred embodiment, in particular, the reciprocal arrangement of the various longitudinal sections detected is determined on the basis of the results of the comparison phase. As was said previously, in the preferred embodiments, the position of the beam 2 can be determined very simply inasmuch as either the beam is at a standstill (if the log turns) or else it turns in a known and controlled way.

Returning to the phase of detection of the surface conformation, in general this can indifferently envisage the detection of the conformation of one or more of the following parts of log 1:
- a part of the side surface 8 of the log 1;
- a part of the surface 16 of one and/or both the extremities of log 1; or
- the entire surface 16 of one and/or both the extremities of the log 1.

The last two variations are the preferred ones in many practical applications.

The figures from 8 to 13 show six possible embodiments for carrying out the scanning of the surface conformation (with reference to the arrangement of figure 1 the figures from 8 to 13 show a view from above of the log 1). Depending on whether the rotation is envisaged of the log 1 or of the beam 2, and therefore depending on the type of movement which the log 1 can undergo, the embodiments most suited to the purpose can be chosen from time to time.

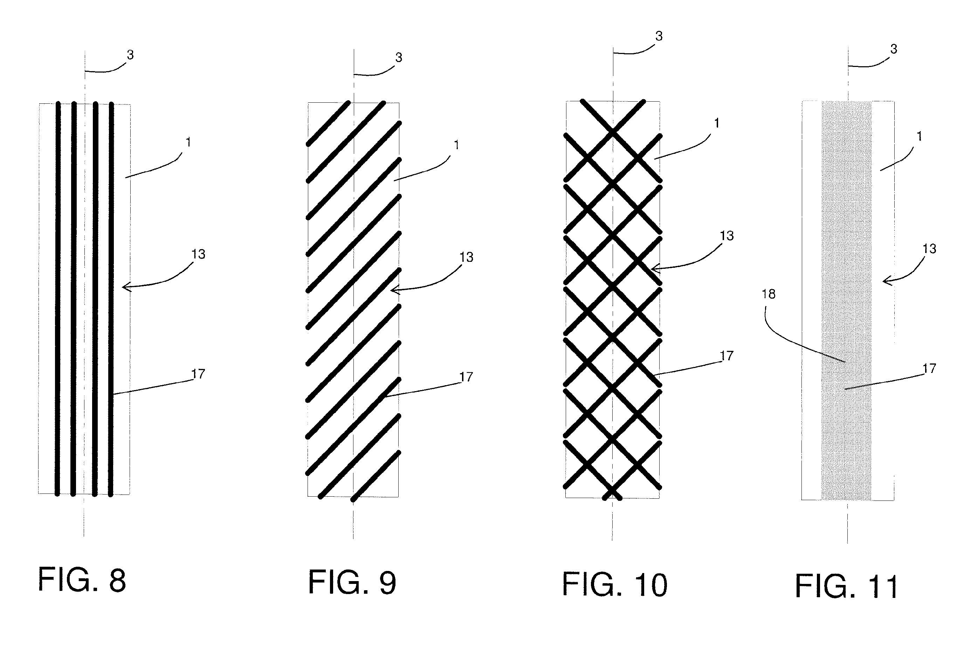

Generally speaking, each scanned surface portion 13 consists of all the linear intersections 17 between the surface of the log 1 and a plurality of distinct surfaces (advantageously flat) affecting this (in the attached figures from 8 to 12 all the planes are perpendicular to the plane of the drawing, but in general these could also be inclined with respect to this). In particular, in the case of the figures 8 and 9 such distinct planes are parallel with one another and, respectively, substantially parallel with the prevalent direction of extension of the log 1 and inclined with respect to this.

In the case shown in figure 10, on the other hand, the surface portion 13 of the scanned side surface 8 of the log 1 consists of the linear intersection 17 between the surface itself and a second plurality of distinct inclined planes, both with respect to it and the one to the other. Such second plurality of planes, in fact, can be split into a first group of planes parallel to one another, the planes of the first group being inclined with respect to the planes of the second group. This way the scanned portion consists of a grid and all the points of the surface are detected several times.

In the case of figure 11, the detection planes are arranged side by side in such a way that each surface portion 13 consists of a longitudinal band 18 of the surface of the log 1.

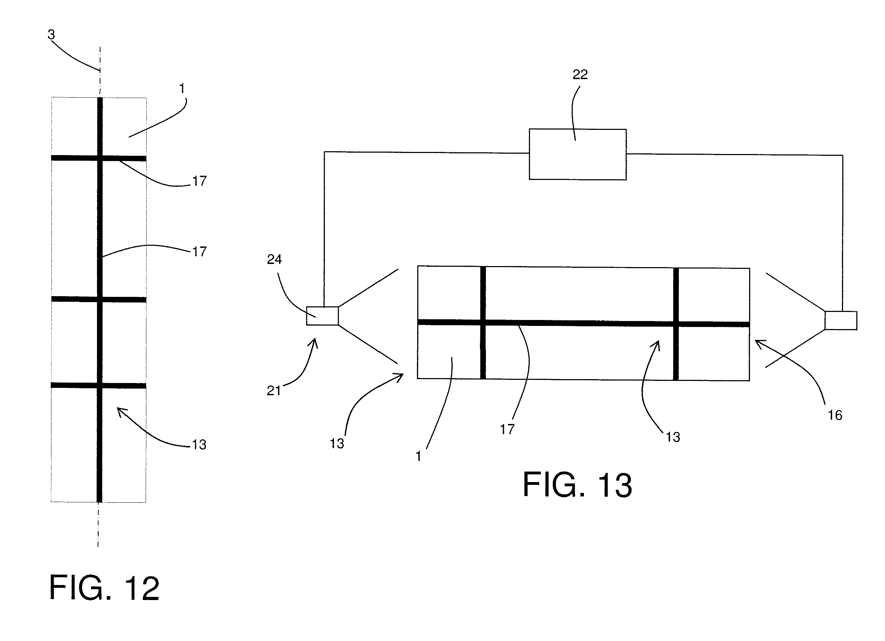

In the case shown in figure 12, the portion of the scanned side surface 8 of the log 1 consists of the linear intersection 17 between the surface itself and one or more first planes substantially parallel to the direction of prevalent extension of the log 1 (or at least arranged in such a way as to affect the entire length of the log 1) and one or more second planes substantially perpendicular to the prevalent direction of extension of the log 1.

Finally, the figure 13 shows a more complex embodiment of the method forming the subject of the present invention which also envisages observing at least an extremity surface 16 of the log 1 (by means of a specific detector 24).

Although the scans of the extremity surface 16 are in this case also associated with a number of scans of the surface conformation of the side surface 8, this is generally not necessary because the appearance of the extremity surface 16 is generally well defined, and the knowledge of this generally allows simplifying the comparison operations described above. Preferably, moreover, for a more accurate assessment of the log 1, it is best to observe both the extremity surfaces 16 considering that the combination of their movements permits describing the required five degrees of freedom of the log 1. Advantageously, to scan the extremity surfaces 16 it is best to use the laser triangulation technique or that tied to the texturing described above.

As has been said above, one of the preferred embodiments of the present invention envisages the use of an electromagnetic beam 2 produced by means of the cone-beam technique.

Especially in this case in fact, in the case of the rotation of the log 1, the method can also comprise a phase of algebraic transformation of the detected movement of the log 1 (with respect to a fixed reference system integral with the supporting and movement means 7) in an apparent movement of the beam 2 of electromagnetic radiations with respect to a reference system integral with the log 1 itself.

This way, in fact, it is always possible to perform the reconstruction phase by means of algorithms based on a processing of the irregular helical movement of the beam around the log, such as those described in the mentioned article M. Kapralov, A. Katsevich - "A Study of 1PI Algorithms for a General Class of Curves", SIAM J. IMAGING SCIENCE, Vol. 1, No. 4, pages 418-459. The apparent movement also in fact configures like an irregular helical movement of the electromagnetic beam 2 with respect to the log 1.

Finally, subject of the present invention is an appliance 9 for scanning the internal quality of logs 1 able to implement the method described thus far. This appliance 9 first of all comprises supporting and movement means 7 of the logs 1 able to move a log 1 forwards along a movement trajectory with its axis arranged substantially parallel to the movement trajectory itself, monitoring its position. It also comprises means of irradiation 19 for irradiating at least a longitudinal section 4 of the log 1 with at least a beam 2 of electromagnetic radiations orientated according to one or more irradiation directions transversal to the main extension axis 3 of a log 1 arranged on supporting and movement means 7. Furthermore, means are envisaged for creating a relative helical movement between the log 1 and the beam 2 of electromagnetic radiations. Depending on requirements, the latter can cause the rotation of either the log 1 or the beam 2 around an instantaneous rotation axis substantially parallel to the movement trajectory. In the preferred embodiment, it is the supporting and movement means 7 that are made in such a way that the log 1 moves with helical movement with respect to the beam 2 of electromagnetic radiations.

The appliance 9 also comprises first detection means 20, comprising at least a detector 5 made up of a plurality of sensors, to detect the residual intensity of the electromagnetic radiations which have crossed the longitudinal section 4 of the irradiated log 1, and second detection means 21 for detecting the spatial position and orientation of the irradiated longitudinal section 4. In particular, in the preferred embodiment, the second detection means detect the surface conformation and the spatial position of at least a surface portion 13 of the log 1.

Finally, the appliance 9 has processing and control means 22 operatively connected to the supporting and movement means 7, to the irradiation means 19, to the first detection means 20 and to the second detection means 21 and programmed to perform the method according to what has been described thus far.

The present invention achieves major advantages.

Firstly, the method forming the subject of the present invention permits performing scans of the internal quality of logs at speeds considerably higher to what has been possible to date, considering it is no longer necessary to precisely control the position of the logs. Every accidental movement of the log can in fact be corrected.

In particular, it makes it possible not to slow down the forward movement of the logs along the machining line.

Furthermore, if the cone-beam technique is used (diverging beam) very long detection sensors can be used with a further increase in work speed.

It must also be pointed out that the present invention is relatively easy to make and that the cost of implementing the invention is not very high.

The invention thus conceived is susceptible to numerous modifications and variations, all falling within the ambit of the inventive concept that distinguishes it.

All the details are replaceable with other technically equivalent elements and in practice, all the materials used, as well as the shapes and the dimensions of the various components, can be of any kind depending on requirements.

## Claims

1. A method for scanning the internal quality of logs, comprising the operating steps of:
taking a log (1) having a main direction of extension identifiable with its main axis of extension (3);
irradiating at least one longitudinal section (4) of the log (1) with at least one beam (2) of electromagnetic radiation in one or more directions of irradiation which are transversal to the main axis of extension (3); and
feeding the log (1) along a movement trajectory with its axis positioned substantially parallel with the movement trajectory, monitoring its position;
during feeding, creating a relative spiral motion between the log (1) and the beam (2) of electromagnetic radiation; during the relative spiral motion and for a plurality of separate reciprocal positions of the log (1) and the beam (2), comprising the operating step of using at least one detector (5) comprising a plurality of sensors to detect the residual intensity of the electromagnetic radiation which passed through the longitudinal section (4) of the log (1);
at least implicitly determining the reciprocal position of the longitudinal sections (4) of the log (1) relative to which said residual intensity of the electromagnetic radiation was detected in the various positions; and reconstructing the internal structure of the log (1) by means of tomographic processing of the residual intensities detected for each position of the log (1);
**characterized in that** the step of feeding the log (1) along the movement trajectory is operated allowing its main axis of extension to freely vary its spatial position perpendicularly to the movement trajectory and/or allowing the advance to experience sliding with respect to a theoretical movement trajectory;
**in that** it further comprises for each detection step an operating step of determining the reciprocal position and spatial orientation of the longitudinal section (4) for which the residual intensity is detected and the beam (2) of electromagnetic radiation with which it was irradiated to take into consideration eventual displacements and/or slides of the log (1);
and also being **characterised in that** the step of reconstructing the internal structure of the log (1) is operated combining the intensities while taking into account the reciprocal positions of the various longitudinal sections (4) relative to each other and relative to the beam (2) of electromagnetic radiation.

2. The method according to claim 1, **characterised in that** the step of creating a relative spiral motion between the log (1) and the beam (2) of electromagnetic radiation occurs either by making the log (1) rotate, or by making the beam (2) of electromagnetic radiation rotate relative to the log (1), about an instantaneous axis of rotation substantially parallel with the movement trajectory.

3. The method according to claim 1 or 2, **characterised in that** the step of determining the reciprocal position and spatial orientation of the longitudinal section (4) and the beam (2) comprises detecting the surface structure and the spatial position of at least one surface portion (13) of the log (1) in such a way that the surface portion (13) detected with each reading is at least partly superimposed on the surface portion (13) detected in a different reading, also being **characterised in that** the step of at least implicitly determining the reciprocal position of the longitudinal sections (4) comprises:
at least implicitly comparing the surface structure detected with a second surface structure detected at a different position of the log (1); and
on the basis of said comparison, at least implicitly determining the reciprocal position of the longitudinal sections of the log (1) relative to which the residual intensity of the electromagnetic radiation was detected in the two positions; and also being **characterised in that** in the step of reconstructing the internal structure of the log (1) by tomographic processing of the residual intensities detected for each position of the log (1), the reciprocal arrangement of the various longitudinal sections detected is determined based on the results of the comparison step.

4. The method according to claim 3, **characterised in that** the comparison step comprises comparing, for each position, the surface structure detected **in that** position with the surface structure detected in a different position, for determining the movements which affected the log (1) as it passed between the two positions and also being **characterised in that** in the reconstructing step the reciprocal arrangement of the various longitudinal sections is calculated based on said movements.

5. The method according to claim 4, **characterised in that** the comparison step comprises comparing, for each position, the surface structure detected **in that** position with the surface structure detected in a plurality of different positions.

6. The method according to any of the claims from 3 to 5, **characterised in that** the step of detecting the surface structure comprises detecting the structure of a part of the lateral surface (8) of the log (1) and/or of a part of and/or the whole surface of one and/or both of the ends of the log (1).

7. The method according to claim 6, **characterised in that** in each step of detecting the surface structure a portion of the surface of the log (1 ) is detected consisting of the linear intersection (17) between the surface and at least one plane incident on it.

8. The method according to claim 7, **characterised in that** in each step of detecting the surface structure a portion of the surface of the log (1) is detected consisting of the linear intersection (17) between the surface and a plurality of separate planes incident on it.

9. The method according to claim 8, **characterised in that** in each step of detecting the surface structure a portion of the surface of the log (1) is detected consisting of the linear intersection (17) between the surface and a first plurality of separate planes which are parallel or substantially parallel with the main direction of extension of the log (1), or which are set at an angle to it.

10. The method according to claim 8, **characterised in that** in each detecting step a portion of the surface of the log (1) is detected consisting of the linear intersection (17) between the surface and a second plurality of separate planes which are set at an angle to it, and which are grouped in a first group and in a second group of planes which are parallel with each other, the planes of the first group being set at an angle to the planes of the second group.

11. The method according to claim 8, **characterised in that** in each detecting step a portion of the surface of the log (1) is detected consisting of the linear intersection (17) between the surface and a first plane substantially parallel with the main direction of extension of the log (1), and at least a second plane substantially perpendicular to the main direction of extension of the log (1).

12. The method according to claim 2, **characterised in that** the step of creating a relative spiral motion between the log (1) and the beam (2) of electromagnetic radiation occurs by making the log (1) rotate and also being **characterised in that** it also comprises a step of algebraic conversion of the motion of the log (1) detected into an apparent motion of the beam (2) of electromagnetic radiation relative to a reference system integral with the log (1), and also being **characterised in that** the reconstructing step is carried out using algorithms based on processing of said apparent motion.

13. The method according to any of the foregoing claims, **characterised in that** during feeding the log (1) is supported at its lateral surface (8).

14. The method according to any of the foregoing claims, **characterised in that** the beam (2) of electromagnetic radiation is a cone-beam style beam (2).

15. An apparatus (9) for scanning the internal quality of logs, comprising:
supporting and movement means (7) for moving a log (1), monitoring its position, along a movement trajectory with its axis positioned substantially parallel with the movement trajectory, but free to vary its spatial position perpendicularly to the movement trajectory and/or free of experiencing sliding with respect to a theoretical movement trajectory;
means (19) for irradiating at least one longitudinal section (4) of the log (1) with at least one beam (2) of electromagnetic radiation in one or more directions of irradiation which are transversal to the main axis of extension (3) of a log (1) positioned on the supporting and moving means (7), the log (1) moving with a spiral motion relative to the beam (2) of electromagnetic radiation;
means for creating a relative spiral motion between the log (1) and the beam (2) of electromagnetic radiation;
first detecting means (20), comprising at least one detector (5) comprising a plurality of sensors for detecting the residual intensity of the electromagnetic radiation which passed through the longitudinal section (4) of the log (1) irradiated;
second detecting means (21) for detecting the reciprocal position and spatial orientation of the longitudinal section (4) irradiated and the beam (2) of electromagnetic radiation; and
processing and control means (22) operatively connected to the supporting and moving means (7), to the irradiating means (19), to the means for creating the spiral motion, to the first detecting means (20) and to the second detecting means (21) and programmed for implementing the method according to any of the foregoing claims.

16. The apparatus according to claim 15, **characterised in that** the second detecting means (21) detect the surface structure and the spatial position of at least one surface portion (13) of the log (1).

17. The apparatus according to claim 15 or 16, **characterised in that** the means for creating the relative spiral motion make the log rotate about an instantaneous axis of rotation which is substantially parallel with the movement trajectory.
